(19) Europäisches Patentamt
European Patent Office
Office européen des brevets

(11) **EP 0 707 500 B1**

(12) **EUROPÄISCHE PATENTSCHRIFT**

(45) Veröffentlichungstag und Bekanntmachung des Hinweises auf die Patenterteilung:
**25.03.1998 Patentblatt 1998/13**

(21) Anmeldenummer: **94921622.0**

(22) Anmeldetag: **23.06.1994**

(51) Int. Cl.$^6$: **A61M 1/10**, F04D 29/22

(86) Internationale Anmeldenummer:
**PCT/EP94/02049**

(87) Internationale Veröffentlichungsnummer:
**WO 95/00185 (05.01.1995 Gazette 1995/02)**

(54) **BLUTPUMPE ALS ZENTRIFUGALPUMPE**

CENTRIFUGAL BLOOD PUMP

POMPE CENTRIFUGE DE SANG

(84) Benannte Vertragsstaaten:
**DE ES FR GB IE IT**

(30) Priorität: **25.06.1993 DE 4321260**

(43) Veröffentlichungstag der Anmeldung:
**24.04.1996 Patentblatt 1996/17**

(73) Patentinhaber:
**BAXTER INTERNATIONAL INC.
Deerfield, Illinois 60015 (US)**

(72) Erfinder:
• **Westphal, Dieter
63128 Dietzenbach (DE)**
• **Reul, Helmut, Prof. Dr.
52353 Düren (DE)**
• **Rau, Günter, Prof. Dr.
52066 Aachen (DE)**

(74) Vertreter:
**Selting, Günther, Dipl.-Ing. et al
Patentanwälte
von Kreisler-Selting-Werner,
Bahnhofsvorplatz 1 (Deichmannhaus)
50667 Köln (DE)**

(56) Entgegenhaltungen:
**EP-A- 0 451 376          US-A- 4 507 048
US-A- 4 589 822**

## Beschreibung

Die Erfindung betrifft eine als Zentrifugalpumpe ausgebildete Blutpumpe mit einem in einem Pumpengehäuse rotierenden Flügelrad.

Eine Blutpumpe, von der der Oberbegriff des Patentanspruchs 1 ausgeht, ist bekannt aus US 4 507 048. Diese Blutpumpe enthält ein Flügelrad, das in dem Pumpengehäuse zwischen zwei Spitzenlagern gelagert ist, wobei die Flügel des Flügelrades an der Vorderseite eines Zentralkonus angeordnet sind. Auf der Rückseite des Zentralkonus befindet sich eine Platte, die einen konstanten Abstand von der Rückwand des Pumpengehäuses hat. Die Flügel sind ähnlich wie Flugzeugflügel profiliert und sie haben einen Umschlingungswinkel von etwa 15°. Die Flügel sind von einem Kegelmantel bedeckt, in dem noch ein weiterer Kegelmantel angeordnet ist, so daß das Flügelrad einen insgesamt rotierenden, teilweise hohlen Körper bildet, in dem die Flügel angeordnet sind.

Aus EP 0 451 376 A1 ist eine Blutpumpe bekannt, bei der das Flügelrad eine ebene Platte aufweist, von der die Flügel nach vorne, zum Einlaß hin, abstehen. Die Flügel sind leicht gebogen und ihre Höhe nimmt linear nach außen hin ab. Das Flügelrad ist an einer einendig in einem Ansatz des Pumpengehäuses gelagerten Welle befestigt. Die Vorderwand des Pumpengehäuses ist kegelstumpfförmig ausgebildet und die Rückwand weicht mit zunehmendem Radius nach hinten zurück.

Ferner ist aus US 4 589 822 eine Blutpumpe bekannt, bei der das Flügelrad an einer Welle befestigt ist, die ebenfalls außerhalb des Pumpengehäuses gelagert ist. Das Flügelrad weist geradlinige Flügel auf, deren Höhe sich nach außen hin linear verringert. Die Vorderwand des Pumpengehäuses ist kegelstumpfförmig ausgebildet und die Rückwand weicht mit zunehmendem Radius zurück. Die Flügel haben nur einen umschlingungswinkel von etwa 60°. Sie überragen die Platte nach außen hin.

Aus US 4 984 972 ist eine Blutpumpe bekannt, bei der ein Flügelrad aus einer Platte mit ebener Oberseite und kegelförmig verlaufender Unterseite auf einem Spitzenlager pendelnd gelagert ist. Die Flügel des Flügelrades fallen radial nach außen linear ab und enden an dem äußeren Plattenrand.

Zentrifugalpumpen für industrielle Anwendungen sind so ausgelegt, daß sie eine hohe Förderrate bei niedrigem Förderdruck liefern. Dagegen müssen Blutpumpen für geringe Fördermengen und relativ hohe Drücke ausgelegt werden. Ein Problem bei Blutpumpen besteht darin, daß sie stark wechselnden Betriebsbedingungen unterliegen, und daß sichergestellt werden muß, daß Blutschädigungen vermieden werden. Eine Blutpumpe wird beispielsweise eingesetzt, um während einer Operation die Pumpfunktion des Herzens eines Patienten zu übernehmen. Wird dem Patient ein gefäßerweiterndes Medikament zugeführt, so nimmt der Flüssigkeitswiderstand des Patientenkörpers ab und der Druck, gegen den die Blutpumpe fördern muß, verringert sich. Ferner können Blutpumpen dazu eingesetzt werden, die Funktion des Herzens voll zu übernehmen oder nur eine das Herz unterstützende Funktion wahrzunehmen. Demnach muß eine Blutpumpe imstande sein, variierende Fördermengen (durch unterschiedliche Drehzahlen) zu liefern. Ferner muß eine Blutpumpe derart ausgebildet sein, daß sie in den vorkommenden weiten Betriebsbereichen blutschädigungsarm arbeitet. Blutschädigungen werden beispielsweise durch örtliche Erwärmungen der Blutpumpe im Lagerbereich des Flügelrades hervorgerufen, vor allem aber durch Schubspannungen und Scherspannungen, denen das Blut in der Zentrifugalpumpe ausgesetzt ist. Derartige Effekte bewirken eine Zersetzung des Bluts durch Hämolyse, wobei Thrombozyten aktiviert werden und aggregieren. Dies kann zu lebensgefährlichen Thrombenbildungen führen. Thrombenbildungen entstehen ferner in Totwassergebieten, in denen das Pumpengehäuse unzureichend durchströmt wird.

Eine Optimierung der Strömungsverhältnisse in Blutpumpen mit dem Ziel der Vermeidung von Blutschädigungen ist wegen der unterschiedlichen Betriebsbedingungen, denen eine Blutpumpe ausgesetzt sein kann, anhand von Berechnungen und theoretischen Überlegungen zur Zeit nicht möglich. Bei der Auslegung einer Blutpumpe ist der Konstrukteur weitgehend auf empirische Forschung angewiesen.

Der Erfindung liegt die Aufgabe zugrunde, eine Blutpumpe zu schaffen, die blutschädigungsarm arbeitet und einfach ausgebildet ist, so daß sie als Einmalartikel kostengünstig herstellbar ist.

Die Lösung dieser Aufgabe erfolgt erfindungsgemäß mit den im Patentanspruch 1 angegebenen Merkmalen.

Bei der erfindungsgemäßen Blutpumpe ist das Flügelrad im Pumpengehäuse zwischen Axialgleitlagern gelagert, wodurch aufwendige Wälzlager und Wellendichtungen entfallen. Dadurch wird die Reibung geringgehalten, so daß eine Reibungserwärmung praktisch nicht feststellbar ist. Der Begriff Axialgleitlager umfaßt Spitzenlager und insbesondere auch ein Kugelspurlager mit einer entlang der Drehachse angeordneten Kugel. Das Flügelrad weist Flügel auf, die von einer Platte frei abstehen und die nicht von einer Bedeckung umschlossen sein sollten. Die einzelnen Flügel können einen sehr hohen Umschlingungswinkel von 90-120°, vorzugsweise von etwa 110°, haben. Infolge des großen Umschlingungswinkels ist die Blutpumpe imstande, den erforderlichen hohen Druck zu liefern und andererseits die Druckerhöhung kontinuierlich und mit geringen Schubspannungen vorzunehmen.

Die Höhe der Flügel nimmt nach außen hin mit zunehmendem Radius linear ab, während die Vorderwand des Pumpengehäuses generell parallel zu der Platte des Flügelrades verläuft, jedoch eine kleine Konizität von etwa 3 bis 10° haben kann, damit bei senkrecht stehender Pumpe Luftblasen zum Einlaß hin entweichen können. Dadurch vergrößert sich der Abstand der Flügel von der vorderwand des Gehäuses linear, so daß der zwischen den Flügeln und der Vorderwand des Gehäuses gebildete Spalt sich linear mit dem Radius vergrößert. Da sich die Umfangsgeschwindigkeit ebenfalls linear mit dem Radius vergrößert, ist die Scherrate an der Vorderseite des Flügelrades im wesentlichen konstant. Es werden also keine sonst üblichen Scherspannungsspitzen erzeugt. An der Rückseite der Platte ist der zwischen der Platte und der Rückwand des Pumpengehäuses gebildete Spalt konstant. Die Spaltbreite sollte größer sein als 1 mm, vorzugsweise etwa 2 mm. Dadurch werden an der Rückseite des Flügelrades Sekundärströmungen erzeugt, in denen das Blut in ständiger Umwälzung gehalten wird, so daß sich keine Totwassergebiete bilden können.

Die Flügel beginnen vorzugsweise an dem Flügelrad erst relativ weit außen, d.h. der von Flügeln freie Mittelbereich hat, bezogen auf den Außendurchmesser des Flügelrades, einen relativ großen Durchmesser. Der Flügeleintrittswinkel beträgt nur 18-25°. Dieser geringe Eintrittswinkel verhindert Scherspannungsspitzen an dem sehr kritischen Eintrittsbereich. Auch der Flügelaustrittswinkel ist vorzugsweise viel kleiner als bei Kreiselpumpen dieser Baugröße üblich.

Im folgenden wird unter Bezugnahme auf die Zeichnungen ein Ausführungsbeispiel der Erfindung näher erläutert.

Es zeigen:

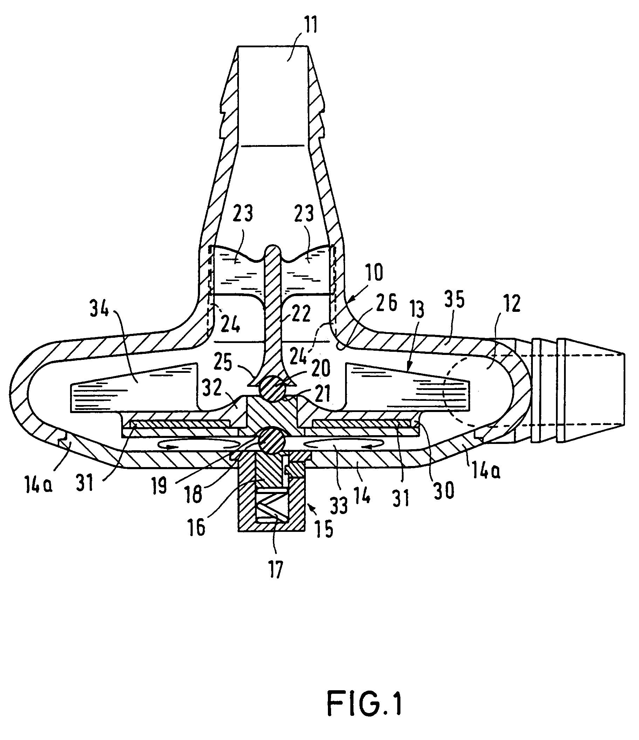

Fig. 1      einen Längsschnitt durch die Blutpumpe,

Fig. 2      eine Ansicht des Flügelrades,

Fig. 3      den Verlauf des Flügelwinkels $\beta$ in Abhängigkeit von dem Radius r,

Fig. 4      einen Längsschnitt durch das Antriebsrad,

Fig. 5      einen Schnitt entlang der Linie V-V von Fig. 4 und

Fig. 6      die Anordnung der weichmagnetischen Platten im Flügelrad.

Die Blutpumpe weist ein rundes, flaches (tellerförmiges) Pumpengehäuse 10 von etwa 60 mm Durchmesser auf, das entlang seiner Achse mit einem Einlaß 11 versehen ist. Der Auslaß 12 ist am Umfang des Pumpengehäuses 10 tangential angeordnet.

Im Pumpengehäuse 10 ist das Flügelrad 13 mit einer Axialgleitlagerung gelagert. In der Rückwand 14 des Pumpengehäuses befindet sich eine Stützvorrichtung 15 für einen Kugelhalter 16. Die Stützvorrichtung 15 enthält eine Feder 17, die den Kugelhalter 16 gegen das Flügelrad 13 drückt. An dem Kugelhalter 16 ist längs der Achse des Flügelrades eine Kugel 18 aus Keramik durch Ankleben befestigt. Diese Kugel 18 ragt in eine Kugelpfanne 19 des Flügelrades 13 hinein, wobei der Durchmesser der Kugelpfanne 19 etwa doppelt so groß ist wie der Durchmesser der Kugel 18.

Als Gegenlager für die Kugel 18 dient eine weitere auf der Flügelradachse angeordnete Kugel 20 aus Keramik, die in eine der Kugelpfanne 19 entgegengerichtete Kugelpfanne 21 eintaucht. Die Kugel 20 ist an einem Kugelhalter 22 durch Ankleben befestigt, der in den rohrförmigen Einlaß 11 hineinragt und mit seitlich abstehenden Rippen 23 versehen ist, die in Längsnuten 24 der rohrförmigen Wand des Einlasses 11 hineinragen. In den beiden Kugelspurlagern beträgt das Verhältnis $R_{Katotte}/R_{Kugel}$ zwischen 1,5/1 und 3/1, so daß die Kugel mit der zugehörigen Kalotte nur Punktkontakt hat. Kugel und Kalotte bestehen aus einer Materialpaarung, die günstige Reibungsbedingungen liefert, z.B. Stahl - Keramik oder Keramik - Keramik.

Das freie Ende 25 des Kugelhalters 22 ist trompetenförmig erweitert, wobei die Erweiterung einen Radius von etwa 5 mm hat. Diese Erweiterung dient dazu, das vom Einlaß 11 kommende Blut radial zum Flügelrad abzulenken. Der Übergang 26 vom Einlaß 11 zum Pumpenraum des Pumpengehäuses hat einen Krümmungsradius von etwa 3 mm. Der Übergang von der axialen Strömung im Einlaß 11 zu der Rotationsströmung im Pumpenraum erfolgt ohne abrupte Querschnittsveränderung. Der Einlaufbereich des Flügelrades, der durch die Strömungsumlenkung um 90° stark beeinflußt wird, ist besonders strömungsgünstig und blutschonend ausgeführt. Dies wird dadurch erreicht, daß beim Übergang vom Einlaß 11 zum Pumpenraum der Strömungsquerschnitt um etwa den Faktor 6 zunimmt (von 140 mm$^2$ im Einlaßstutzen auf 850 mm$^2$ am Flügelbeginn), und daß andererseits die Umlenkradien an dieser Stelle besonders groß sind.

Das Flügelrad 13 weist eine ebene Platte 30 auf, in der ferromagnetische Platten 31 eingebettet sind. Die Platte 30 ist in ihrem Mittelbereich 32 an der dem Einlaß 11 zugewandten Seite um weniger als die Plattenstärke verdickt ausgebildet. Diese verdickung im Mittelbereich 32 ist abgerundet, wobei der Radius demjenigen des trompetenförmigen Bereichs 25 entspricht. Der Mittelbereich 32 ist von Flügeln frei. Sein Radius R1 beträgt 9 mm und ist etwas größer als der Radius des Einlasses 11 am Übergang in die Pumpenkammer. Der Radius R2 des Flügelrades beträgt 24 mm und der Radius R3 der Platte 30 beträgt 18 mm.

Zwischen der Rückseite der Platte 30 und der Rückwand 14 ist ein Spalt 33 konstanter Breite von etwa 2 mm gebildet. Bei Rotation des Flügelrades 13 bilden sich in dem Spalt 33 Sekundärströmungen aus, die durch die Pfeile angedeutet sind. Diese Sekundärströmungen verhindern die Bildung von Totwasserbereichen in dem Spalt 33.

Das Flügelrad 13 weist vier bis sieben (hier: fünf) Flügel 34 auf, die am Umfang des Mittelbereichs 32 beginnen und mit etwa einem Drittel der Flügellänge radial über die Platte 30 hinausragen. Die Rückwand 14 ist im Bereich der Überstände der Flügel 34 mit einer Abschrägung 14a versehen, um das Fehlen der Platte 30 im äußeren Bereich der Pumpenkammer zu kompensieren. Die Weite der Pumpenkammer verringert sich somit zum äußeren Rand und zum Auslaß 12 hin.

Die vordere Wand 35 der Pumpenkammer verläuft annähernd parallel zu dem mittleren Teil der Rückwand 14 und zu der Platte 30. Die Flügel 34 haben ihre größte Höhe am inneren Ende, d.h. am Umfang des Mittelbereichs 32. Die Flügelhöhe verringert sich linear nach außen hin auf etwa die Hälfte. Der Spalt zwischen der Vorderwand 35 des Pumpengehäuses und den Flügelkanten verbreitert sich radial nach außen. Da sich die Umfangsgeschwindigkeit des Blutes ebenfalls radial nach außen vergrößert, bleibt die Scherrate konstant.

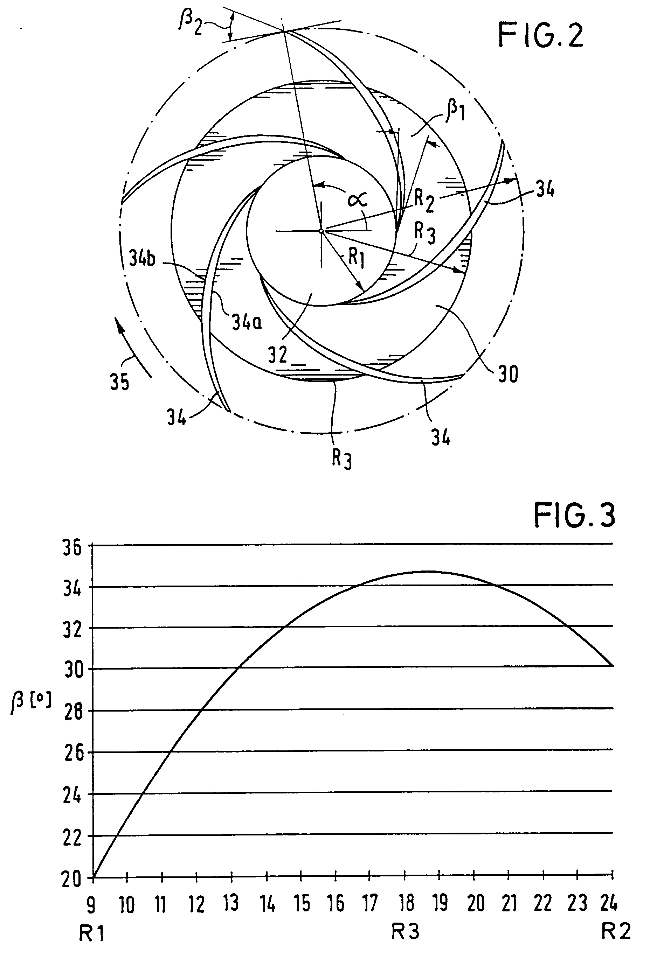

Wie Fig. 2 zeigt, ist jeder Flügel 34 in Rotationsrichtung 35 bogenförmig gekrümmt, wobei der Umschlingungswinkel α eines Flügels 100° beträgt. Der Flügeleintrittswinkel β1, nämlich der Tangentenwinkel des Flügels an den Mittelbereich 32, beträgt 20° und der Flügelaustrittswinkel β2, nämlich der Tangentenwinkel des Flügels an den die Flügelenden umschließenden Kreis mit dem Radius R2, beträgt 30°. Die konkave Innenseite 34a des Flügels bildet die Saugseite und die konvexe Außenseite 34b bildet die Druckseite. Am Eintrittsende und am Austrittsende haben die Flügel ihre geringste Dicke. Die Flügeldicke vergrößert sich aus Festigkeitsgründen zur Mitte hin, wobei der Flügel im Mittelbereich die größte Dicke hat. Am Eintrittsende ist der Flügel abgerundet.

Fig. 3 zeigt den Verlauf des Tangentenwinkels β des Flügels in Abhängigkeit von dem Radius r. Der Verlauf β(r) entspricht einem Polynom zweiten Grades:

$$\beta(r) = Ar^2 + Br + C.$$

Hierbei sind A, B und C Konstanten. Bei dem vorliegenden Ausführungsbeispiel ist A = -0,16; B = 5,95; C = -20,56.

Die beschriebene Bauform von Pumpengehäuse und Flügelrad ermöglicht bei gleicher Förderleistung ein wesentlich geringeres Füllvolumen als andere Blutpumpen. Das Füllvolumen beträgt hier nur 30 ml. Durch das geringe Füllvolumen wird das extrakorporale Blutvolumen verringert und der Kontakt des Blutes mit Fremdflächen, der erheblich zur Blutschädigung beiträgt, reduziert. Die Blutpumpe kann aus einer geringen Zahl von Teilen kostengünstig hergestellt werden. Aus medizinischen Gründen ist sie für den Einmalgebrauch vorgesehen.

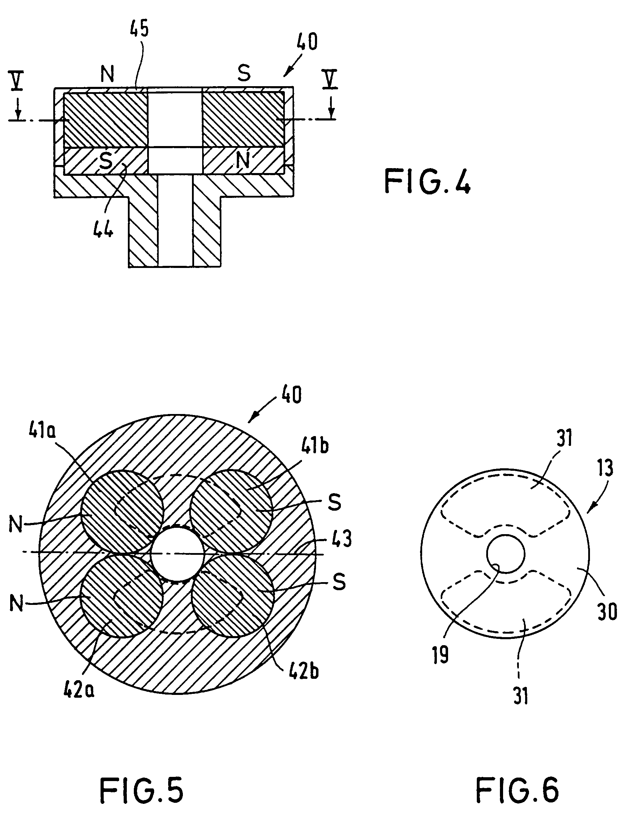

Die beschriebene Blutpumpe wird in Verbindung mit dem in den Fign. 4 und 5 dargestellten Antriebsrad 40 benutzt. Dieses Antriebsrad weist zwei Paare 41a,41b;42a,42b von Stabmagneten auf, wobei jedes Paar auf einer anderen Seite des Radius 43 des Antriebsrades angeordnet ist. In den Zeichnungen sind die Polungen der Stabmagnete mit N (Nordpol) und S (Südpol) angegeben. Die Rückseiten der Stabmagnete sind mit einer ferromagnetischen Platte 44 in Kontakt, die den Polrückschluß bildet. Die Vorderseite 45 der Stabmagnete wirkt durch die Wand des Pumpengehäuses 10 hindurch magnetisch auf die ferromagnetischen Platten 31 ein, die den Magnetfluß auf der Vorderseite der Stabmagnete schließen. Das Antriebsrad 40 wird von einem Motor angetrieben und nimmt dabei die im Pumpengehäuse 10 gelagerte Scheibe 30 mit. Die in der Scheibe 30 enthaltenen Platten 31 bestehen lediglich aus weichmagnetischem (ferromagnetischem) Material, z.B. aus einfachem Baustahl. Sie haben jeweils eine Umfangserstreckung von 120° und eine Dicke von 1 mm.

Wenn die Pumpe mit einer Drehzahl von 3.000 U/min angetrieben wird, liefert sie eine Fördermenge von 4 l/min bei einem Förderdruck von 180 mmHg.

**Patentansprüche**

1. Blutpumpe als Zentrifugalpumpe, mit einem Pumpengehäuse (10), das einen axialen Einlaß (11) und einen an seinem Umfang angeordneten Auslaß (12) aufweist, und einem in dem Pumpengehäuse (10) zwischen zwei Axialgleitlagern gelagerten Flügelrad (13), das ferromagnetische Kopplungsteile (31) enthält und an seiner dem Einlaß (11) zugewandten Vorderseite von einer ebenen Platte (30) abstehende Flügel (34) aufweist, deren Höhe radial nach außen abnimmt, wobei die ebene Rückseite der Platte (30) einen im wesentlichen konstanten Abstand von der Rückwand (14) des Pumpengehäuses (10) hat,
   **dadurch gekennzeichnet,**

   daß die Höhe der Flügel (34) mit zunehmendem Radius linear abnimmt und die Vorderwand (35) des Pumpengehäuses (10) im wesentlichen parallel oder mit einer kleinen Konizität von etwa 3 bis 10° zu der Platte (30) des Flügelrades (13) verläuft, und

   daß die Flügel (34) die Platte (30) nach außen hin überragen und die Rückwand (14) des Pumpengehäuses außerhalb der Platte (30) eine umlaufende Abschrägung (14a) aufweist, die einen kontinuierlichen Übergang zu dem tangentialen Auslaß (12) bewirkt.

2. Blutpumpe nach Anspruch 1, dadurch gekennzeichnet, daß der dem Einlaß (11) zugewandte Mittelbereich (32) der Platte (30) um weniger als die Plattenstärke verdickt ausgebildet ist, und daß die Flügel (34) angrenzend an den Mittelbereich (32) beginnen.

3. Blutpumpe nach Anspruch 1 oder 2, dadurch gekennzeichnet, daß jeder Flügel (34) sich über einen Umschlingungswinkel von 90° bis 120° erstreckt.

4. Blutpumpe nach einem der Ansprüche 1-3, dadurch gekennzeichnet, daß der Flügeleintrittswinkel (β1), d.h. der Tangentenwinkel der Flügel an den Mittelbereich (32) der Platte (30) 18-25° beträgt.

5. Blutpumpe nach einem der Ansprüche 1-4, dadurch gekennzeichnet, daß der Flügelaustrittswinkel (β2), d.h. der Tangentenwinkel der Flügel (34) an einen die Flügelenden umschließenden Kreis 25-40° beträgt.

6. Blutpumpe nach einem der Ansprüche 1-5, dadurch gekennzeichnet, daß der Radius R1 des von den Flügeln (34) freien Mittelbereichs (32) der Platte (30) zu dem Radius R2 eines die Flügelenden umschließenden Kreises im Verhältnis R1:R2 von 0,25-0,5 steht.

7. Blutpumpe nach einem der Ansprüche 1-6, dadurch gekennzeichnet, daß die Flügelstärke vom inneren Ende eines Flügels (34) nach außen hin zunimmt und anschließend wieder abnimmt.

8. Blutpumpe nach einem der Ansprüche 1-7, dadurch gekennzeichnet, daß der Flügelwinkel β, d.h. der Tangentenwinkel der Skelettlinie eines Flügels, einem Polynom zweiten Grades $Ar^2 + Br + C$ entspricht, wobei r der Radius und A, B und C Konstanten sind.

9. Blutpumpe nach einem der Ansprüche 1-8, dadurch gekennzeichnet, daß die Axialgleitlager jeweils eine Kugel (18,20) aufweisen, die in eine Kugelpfanne (19,21) der Platte (30) eingreift, wobei der Durchmesser der Kugelpfanne größer ist als derjenige der Kugel.

10. Blutpumpe nach einem der Ansprüche 1-9, dadurch gekennzeichnet, daß das Flügelrad (13) zwei weichmagnetische Platten (31) enthält, und daß ein Antriebsrad (40) vorgesehen ist, das zwei Paare von Stabmagneten (41a,41b;42a,42b) enthält, von denen jedes auf einer anderen Seite des Durchmessers (43) des Antriebsrades angeordnet ist.

**Claims**

1. A blood pump as centrifugal pump, comprising a pump housing (10) having an axial inlet (11) and an outlet (12) arranged on its periphery, and an impeller (13) supported in said housing (10) between two sliding thrust bearings and comprising ferromagnetic coupling members (31) and blades (34) projecting from a plane plate (30) at its front side facing said inlet (11), the height of which blades decreases radially outwards, the plane back side of said plate (30) having a substantially constant distance from the back wall (14) of said pump housing (10),

**characterized in**

that the height of said blades (34), with increasing radius, decreases linearly, and the front wall (35) of said pump housing (10) extends substantially parallel or with a small conicity of approximately 3 to 10° to said plate (30) of said impeller (13), and

that the blades (34) project outwards beyond the plate (30), and the back wall (14) of the pump housing comprises, outside the plate (30), a circumferential sloping (14a) causing a continuous transition to the tangential outlet (12).

2. The blood pump according to claim 1, characterized in that the central portion (32) of said plate (30), which faces said inlet (11), is thickened by less than the plate thickness, and that said blades (34) begin adjacent to said central portion (32).

3. The blood pump according to claim 1 or 2, characterized in that each blade (34) extends over a circumferential angle of 90° to 120°.

4. The blood pump according to one of claims 1-3, characterized in that the blade inlet angle (β1), i.e. the tangent angle of said blades to said central portion (32) of said plate (30), amounts to 18-25°.

5. The blood pump according to one of claims 1-4, characterized in that the blade outlet angle (β2), i.e. the tangent angle of said blades (34) to a circle encompassing the blade ends, amounts to 25-40°.

6. The blood pump according to one of claims 1-5, characterized in that the ratio of R1/R2 between the radius R1 of said central portion (32) of said plate (30), which is free of blades (34), and the radius R2 of a circle encompassing the blade ends is 0.25-0.5.

7. The blood pump according to one of claims 1-6, characterized in that the blade thickness increases from the inner end of a blade (34) and subsequently decreases again.

8. The blood pump according to one of claims 1-7, characterized in that said blade angle β, i.e. the tangent angle of the median line of a blade, corresponds to a polynomial of the second degree $Ar^2 + Br + C$, wherein r is the radius and A, B, and C are constants.

9. The blood pump according to one of claims 1-8, characterized in that the sliding thrust bearings each comprise a ball (18,20) which engages into a ball cup (19,21) of said plate (30), the diameter of said ball cup being larger than that of said ball.

10. The blood pump according to one of claims 1-9, characterized in that said impeller (13) includes two soft magnetic plates (31), and that a driving wheel (40) is provided which includes two pairs of bar magnets (41a,41b;42a,42b), each of which is arranged on a different side of the diameter (43) of said driving wheel.

**Revendications**

1. Pompe à sang sous forme de pompe centrifuge, comportant un boîtier de pompe (10) qui présente une entrée axiale (11) et une sortie (12) disposée sur sa périphérie, ainsi qu'une roue à ailettes (13) qui est disposée, dans le boîtier de pompe (10), entre deux paliers formant butées axiales, qui contient des éléments ferromagnétiques de couplage (31) et qui présente, sur sa face avant, située du côté de l'entrée (11), des ailettes (34) en saillie à partir d'une plaque plane (30), ailettes dont la hauteur va en décroissant radialement vers l'extérieur, la face arrière plane de la plaque (30) étant à une distance sensiblement constante de la paroi arrière (14) du boîtier de pompe (10),
caractérisée par le fait que la hauteur des ailettes (34) décroît linéairement quand le rayon croît et que la paroi avant (35) du boîtier de pompe (10) est orientée sensiblement parallèlement à la plaque (30), ou avec une faible conicité d'environ 3 à 10° par rapport à cette plaque, et
que les ailettes (34) débordent de la plaque (30) vers l'extérieur et que la paroi arrière (14) du boîtier de pompe présente, à l'extérieur de la plaque (30), un biseau périphérique (14a) qui réalise une transition continue jusqu'à la sortie tangentielle (12).

2. Pompe à sang selon la revendication 1, caractérisée par le fait que la zone médiane (32) de la plaque (30), qui est située du côté de l'entrée (11), est prévue avec un épaississement inférieur à l'épaisseur de la plaque et que les ailettes (34) commencent en jouxtant la zone médiane (32).

3. Pompe à sang selon la revendication 1 ou 2, caractérisée par le fait que chaque ailette (34) s'étend sur un angle au centre de 90° à 120°.

4. Pompe à sang selon l'une des revendications 1-3, caractérisée par le fait que l'angle d'entrée (β1) de l'ailette, c'est-à-dire l'angle que fait l'ailette avec la tangente à la zone médiane (32) de la plaque (30), vaut 18-25°.

5. Pompe à sang selon l'une des revendications 1-4, caractérisée par le fait que l'angle de sortie (β2) de l'ailette, c'est-à-dire l'angle que fait l'ailette (34) avec la tangente à un cercle circonscrit aux extrémités des ailettes vaut 25-40°.

6. Pompe à sang selon l'une des revendications 1-5, caractérisée par le fait que le rayon (R1) de la zone médiane (32) de la plaque (30), qui est exempte des ailettes (34), et le rayon (R2) d'un cercle circonscrit aux extrémités des ailettes sont dans un rapport R1:R2 de 0,25-0,5.

7. Pompe à sang selon l'une des revendications 1-6, caractérisée par le fait que l'épaisseur de l'ailette croît de l'extrémité intérieure d'une ailette (34) vers l'extérieur puis décroît ensuite.

8. Pompe à sang selon l'une des revendications 1-7, caractérisée par le fait que l'angle β de l'ailette, c'est-à-dire l'angle que fait avec la tangente la ligne médiane d'une ailette, correspond à un polynôme du second degré $Ar^2 + Br + C$, r étant le rayon et A, B et C étant des constantes.

9. Pompe à sang selon l'une des revendications 1-8, caractérisée par le fait que les paliers formant butées axiales présentent chacun une bille (18, 20), qui se loge dans un logement sphérique (19, 21) de la plaque (30), le diamètre du logement sphérique étant supérieur à celui de la bille.

10. Pompe à sang selon l'une des revendications 1-9, caractérisée par le fait que la roue à ailettes (13) contient deux plaquettes (31) à aimantation douce et qu'est prévue une roue d'entraînement (40) qui contient deux paires de barreaux aimantés (41a, 41b ; 42a, 42b) dont chacune est disposée d'un côté différent du diamètre (43) de la roue d'entraînement.

FIG.1

FIG.2

FIG.3

FIG.4

FIG.5

FIG.6